# EUROPEAN PATENT SPECIFICATION

(11) **EP 0 644 887 B1**
(45) Date of publication and mention of the grant of the patent: **11.12.1996**
(21) Application number: 93913311.2
(22) Date of filing: 07.06.1993
(51) Int. Cl.: C07D 503/00

(54) **INTERMEDIATES IN A PROCESS FOR THE PURIFICATION OF CLAVULANIC ACID**
ZWISCHENPRODUKTE IN EINEM VERFAHREN ZUR REINIGUNG VON CLAVULANSÄURE
INTERMEDIAIRES D'UN PROCEDE POUR LA PURIFICATION DE L'ACIDE CLAVULANIQUE

(30) Priority: 11.06.1992 GB 9212379; 31.10.1992 GB 9222841; 14.12.1992 GB 9226061; 17.12.1992 GB 9226282
(43) Date of publication of application: 29.03.1995
(60) Divisional application: 95201084.1
(73) Proprietor: SMITHKLINE BEECHAM PLC, Brentford, Middlesex TW8 9EP (GB)
(72) Inventor: COOK, Michael Allen, Worthing, West Sussex BN14 8QH (GB); WILKINS, Robert Bennett, Irvine, Ayrshire KA11 5AP (GB)
(74) Representative: Lawton, Peter Philip
(86) International application number: GB9301206
(87) International publication number: WO9325557

(56) References cited:
- EP-A- 0 026 044
- EP-A- 0 312 813
- EP-A- 0 387 178
- GB-A- 1 578 739

## Description

This invention relates to a novel process for the preparation of clavulanic acid (I): 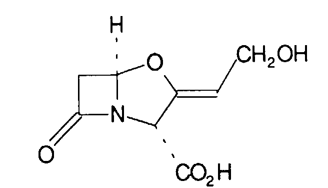 and pharmaceutically acceptable salts and esters thereof.

Clavulanic acid is normally prepared by the fermentation of a microorganism which produces clavulanic acid, such as various microorganisms belonging to various *Streptomyces* strains such as *S. clavuligerus* NRRL 3585, *S. jumoninensis* NRRL 5741, *S. katsurahamanus* IFO 13716 and *Streptomyces* sp. P 6621 FERM P2804 e.g. as described in JP Kokai 80-162993. The resulting aqueous broth may be subjected to conventional purification and concentration processes, for example involving filtration and chromatographic purification, such as disclosed in GB 1508977 and JP Kokai 80-62993, before extraction of the aqueous solution with an organic solvent to yield a solution of crude clavulanic acid in the organic solvent.

GB 1508977 discloses inter alia that salts of clavulanic acid can be obtained by absorbing the clavulanate anion in filtered broth on to an anion exchange resin, eluting therefrom with an electrolyte, desalting the resulting solution, applying the desalted solution to a further anion exchange resin, chromatographically eluting therefrom with an electrolyte, desalting the resulting solution and thereafter removing the solvent. This process can be used to give acceptable yields of pure material but the use of resin columns involves significant investment and they can introduce limitations in large scale production operations. It would therefore be desirable to have an alternative procedure available that involved few resin utilizing stages.

GB 1543563 discloses a process for the preparation of clavulanic acid salts via precipitation of lithium clavulanate. GB 1578739 describes various amine salts of clavulanic acid as pharmaceutical compounds. EP 0026044 discloses the use of the tertiary-butylamine salt of clavulanic acid as a useful intermediate in the preparation of clavulanic acid. The salt has been disclosed in BE 862211, but only as a suitable ingredient for pharmaceutical formulations. PT.94.908 describes the use of tri-(lower alkyl)amine salts and the dimethylaniline salts of clavulanic acid in a purification process for clavulanic acid in which the triethylamine salt of clavulanic acid is formed and is then converted into a silyl diester of clavulanic acid. EP 0887178A discloses a process for the purification of clavulanic acid in which organic amines may be used to form an intermediate amine salt with clavulanic acid in an impure solution.

The present invention provides salts of clavulanic acid with polymines of the general formula (IIa) as a solid precipitate. 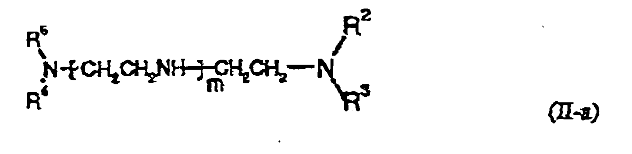 wherein R⁴ and R⁵ are independently hydrogen C₁₋₆alkyl, amino-substatuted C₁₋₆ alkyl or substituted amino-substituted C_{1-6,} alkyl and R² and R³ are independently selected from hydrogen, C₁₋₆ alkyl, amino or hydroxy substituted C₁₋₆ alkyl or substituted amino-substituted C₁₋₆ alkyl and m is zero or an integer 1 to 5 provided that when m is zero alkylenediammonium diclavulanates with the exclusion of compounds, wherein R⁴ and R⁵ are hydrogen, C₁₋₆ alkyl or an aminoalkyl group having 2 to 4 carbon atoms, which is optionally substituted by an N-alkyl or N,N-dialkyl group having 1 to 4 carbon atoms and wherein R² and R³ are hydrogen, C₁₋₆ alkyl, hydroxyalkyl group having 2 to 4 carbon atoms, which is optionally substituted by an N-alkyl or N,N-dialkyl group having 1 to 4 carbon.

Typically, this invention also provides a process for preparing potassium clavulanate which comprises converting a salt of this invention, as above, to potassium clavulanate.

When alkyl groups or substituted alkyl groups are referred to herein unless otherwise defined herein they may suitably contain 1 to 6 carbon atoms in the alkyl system. Suitable substituents on amino groups include alkyl.

R⁴ and R⁵ may suitably both be hydrogen, or one may be hydrogen and the other alkyl. R² and R³ may suitably be hydrogen or alkyl.

Examples of such amines include ethylene diamine, NN-diethylethylene diamine, NN'-diisopropylethylenediamine and triethylene tetramine.

The amine (II-a) may form a salt with one or more of the nitrogen atoms, for example as in NN'-diisopropylethylenediamine diclavulanate.

Of the amines mentioned above a preferred amine is: diisopropylethylenediamine.

WO-A-93/25557 discloses a process for the preparation and/or purification of clavulanic acid or a pharmaceutically acceptable salt or ester thereof which process comprises:
i) contacting impure clavulanic acid or a labile derivative thereof in solution in an organic solvent, with an amine of the formula (II) or a labile derivative thereof,
ii) isolating the salt of clavulanic acid formed with the amine of formula (II), and,
iii) converting the thus formed salt into clavulanic acid or a pharmaceutically acceptable salt or ester thereof.

Salts of the present invention of clavulanic acid with the amine (II-a) may be used to purify impure clavulanic acid during the above preparation. Therefore the salt may be formed in a solution of clavulanic acid or a labile derivative thereof containing impurities, isolating the salt as a separate phase, eg as a solid precipitate, from the solution containing residual impurities, then reforming clavulanic acid or forming a pharmaceutically acceptable salt or ester thereof.

Suitable labile derivatives of clavulanic acid include salts, eg an alkali metal salt such as lithium or sodium clavulanate or esters, such as silyl esters. Suitable labile derivatives of the amine (II) include salts such as the phosphate, borate, chloride, chlorate, perchlorate, bromide, toluene sulphonate or alkanoates, such as the acetate or ethylhexonoate. Conveniently the amine (II) itself is contacted with impure clavulanic acid itself in solution in an organic solvent.

The above process is suitably carried out in an organic solvent, which although preferably substantially dry, for example containing less than 6 g/L, eg 0.25-0.6 g/L of water, may contain some water, as a solvent for the clavulanic acid and the amine (II). A suitable degree of dryness may be achieved by conventional dewatering processes such as centrifuging. Water present in the solvent may be dissolved or in the form of droplets of a separate phase.

The solution of clavulanic acid in organic solvent may be obtained by extraction of an acidified aqueous solution of clavulanic acid such as the fermentation liquor referred to above. If the initial source of the clavulanic acid is a broth resulting from fermentation of a clavulanic acid-producing microorganism, such as those mentioned above, then to obtain a solvent extract of a suitable concentration of clavulanic acid for use in this process it may be desirable not to extract the broth itself, but to at least remove some of the suspended solids in the broth, e.g by filtration prior to extraction. It may also be desirable in addition to pre-concentrate the aqueous solution of clavulanic acid obtained in fermentation, so that for example the aqueous solution of clavulanic acid is several times more concentrated in clavulanic acid than the starting broth, for example pre-concentrated to a concentration of ca. 10 - 100 mg/ml. e.g 10 - 40 mg/ml, such as 10 - 25 g/L clavulanic acid.

Suitable pre-concentration processes include absorption of the clavulanic acid onto an anion exchange resin, followed by elution of the clavulanic acid therefrom with an aqueous solution of an electrolyte such as sodium chloride, and optionally desalting. It is also preferred to acidify the aqueous solution, e.g the broth or the pre-concentrated aqueous solution prior to solvent extraction, e.g to pH 1 to 3, e.g around pH 1.5 to 2.5. It is also preferred to dry or de-water the organic solvent extract prior to formation of the salt with the amine (II-a), e.g to less than 6g/L of water. Preferably the extraction is carried out at a temperature from 5 to 15°C.

Suitable organic solvents in which impure clavulanic acid may be contacted with the amine (II-a) include hydrocarbon solvents such as toluene and hexane, ethers such as tetrahydrofuran, dioxan, diethyl ether, halogenated solvents such as dichloromethane and chloroform, ketones such as acetone and methyl isobutyl ketone, and esters such as ethyl acetate. Solvents which include a carbonyl group, eg those of the formula (III): 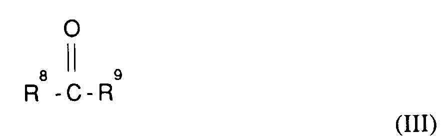 wherein R⁸ is a C₁₋₆ alkyl group or a C₁₋₆ alkoxy group and R⁹ is a C₁₋₆ alkyl group are examples of a sub-class of suitable solvents, for example organic ketones or organic alkanoate esters. The present invention also encompasses the use of mixtures of such solvents.

More suitably the organic solvent is one which can be used directly to extract the acidified aqueous for example organic alkyl alkanoate esters, ketones and certain aliphatic alcohols, or mixtures thereof, such as ethyl acetate, methyl acetate, propyl acetate, n-butyl acetate, methyl ethyl ketone, methyl isobutyl ketone, tetrahydrofuran, butanol and mixtures of such solvents. Of these the most suitable appear to be methyl isobutyl ketone, methyl ethyl ketone, and ethyl acetate. Suitable solvent mixtures include methyl ethyl ketone/methyl isobutyl ketone and tetrahydrofuran/methyl isobutyl ketone. A preferred solvent is ethyl acetate.

Suitable solvents for the amine (II-a) include those referred to above in which the clavulanic acid may be dissolved, or extracted, for example acetone, ethyl acetate, methyl isobutyl ketone, and methyl ethyl ketone.

It appears to be particularly desirable to include ketones such as acetone in the solvent system, as these appear to inhibit the formation of the salt of clavulanic acid with the amine (II-a) as an oil.

In general one equivalent of the amine (II-a) or a slight excess thereof per mole of clavulanic acid is used to produce the salt of clavulanic acid. Solutions of clavulanic acid and amine (II-a) may for example be mixed slowly with stirring and the mixture stirred for some time after addition is complete. The reaction between the clavulanic acid or its labile derivative is suitably carried out at a temperature below ambient, for example 0 to 15°C, eg 0 to 10°C, eg 0 to 5°C. A suitable concentration for the clavulanic acid or its labile derivative in the solution is at least 1.0 g/L, for example in the range 1.0 to 4.0 g/L of clavulanic acid. It may be advantageous to further concentrate the solvent extract to a concentration in excess of this eg greater than 20g/L.

For example in another procedure the amine (II-a) may be introduced by mixing it into a stream of a solution of the clavulanic acid in the solvent, so that the salt is formed in the stream, either in solution or as particles or suspended droplets of the dissolved salt in suspension. The amine (II-a) introduced in this way may be introduced neat, or may be introduced as a solution in a solvent, for example the same organic solvent as the clavulanic acid is dissolved in.

The desired salt of clavulanic acid with the amine (II-a) may then be isolated. In this way, the salt of clavulanic acid with the amine (II-a) is separated from most or all of the impurities. Isolation may be effected in a conventional manner, for example by centrifugation or filtration.

In an alternative isolation procedure the salt of clavulanic acid with the amine (II-a) may be isolated from the organic solvent, if the solvent is wholly or partly immiscible with water, by contacting the solvent with water so as to extract the salt, which may be in solution or suspension, from the organic solvent and to form an aqueous solution of the salt. As salts of clavulanic acid with the amine (II-a) are fairly soluble in water, such an aqueous solution may be very concentrated, eg around 20-30% w:w.

In this way the bulk of organic impurities in the organic solvent solution of clavulanic acid remain in the organic solvent whilst the clavulanic acid, in the form of its salt with the amine (II-a), in a relatively pure state is obtained in the aqueous solution. The aqueous solution of the clavulanic acid salt so formed may be subjected to conventional further treatment, eg purification, or conversion into clavulanic acid or a pharmaceutically acceptable salt or ester as described below.

In another alternative or additional procedure the clavulanic acid and the amine may be mixed in solution in a first organic solvent, then the salt may be caused to separate from solution by addition of a second organic solvent. Suitably the first organic solvent may be an organic ester such as ethyl acetate, and the second solvent may for example be a halogenated solvent such as chloroform, an ether such as diethyl ether, a hydrocarbon such as toluene, an alcohol such as ethanol, or a solvent of formula (III) above such as acetone or methyl isobutyl ketone.

In one embodiment of this invention, the salt of clavulanic acid with the amine (II-a) may be employed as an acetone solvate. Acetone solvates in some cases have advantageous stability and purity characteristics compared to salts of clavulanic acid with amines themselves. Such solvates are particularly useful in the present invention because they can often be isolated as a highly pure and stable crystalline compound.

Accordingly the present invention also provides the salt of clavulanic acid with the amine (II-a) in the form of an acetone solvate. During isolation and/or drying, some acetone may be lost since the strength of solvation may not be high, but the amount of acetone in the product is not critical.

The acetone solvate may be formed by contacting clavulanic acid with the amine (II-a) in the presence of acetone. In general, a solution containing clavulanic acid may be mixed with at least the same volume of acetone together with the amine (II-a), when the salt is precipitated.

The amine (II-a) may be dissolved in acetone and mixed with a solution of clavulanic acid in an organic solvent. Favoured organic solvents include ethyl acetate, tetrahydrofuran, methyl ethyl ketone, methyl isobutyl ketone and mixtures of such solvents, of which ethyl acetate is preferred. Alternatively an aqueous solution of the salt of clavulanic acid with the amine (II-a), obtained by water extraction as outlined above, may be mixed with acetone to form the solvate. Suitably a concentrated aqueous solution of the salt may be mixed with excess acetone to form the solvate.

Recrystallisation of the salt of clavulanic acid or the acetone solvate may be advantageous to further reduce the level of impurities. A convenient solvent for the recystallisation is aqueous acetone. Such recrystallisation is performed in a conventional manner, for example the salt or solvate is dissolved in water, treated with a small amount of acetone, filtered, and then treated with larger volumes of acetone optionally with stirring and/or cooling to afford the recrystallised product.

The salts of clavulanic acid with an amine of formula (II-a) may be converted into clavulanic acid or a pharmaceutically acceptable salt or ester thereof.

The pharmaceutically acceptable salts and esters of clavulanic acid thus prepared include those described in GB 1508977 and 1508978 which are herein incorporated by reference.

Particularly suitable pharmaceutically acceptable salts include the pharmaceutically acceptable alkali and alkaline earth metal salts, for example the sodium, potassium, calcium and magnesium salts. Of these salts the sodium and potassium are most suitable and the potassium is preferred.

Suitable esters include those cleavable to provide clavulanic acid or a salt thereof, by chemical methods such as hydrogenolysis or by biological methods.

The salt of clavulanic acid with amine (II-a) optionally in the form of its acetone solvate may be converted into clavulanic acid or a pharmaceutically acceptable salt or ester thereof by for example ion-replacement in the case of the free acid or salts, or by esterification.

Ion-replacement may be performed using ion-exchange resins, for example by passing a solution of the salt through a bed of a cation exchange resin in sodium, potassium or calcium form. Suitable cation exchange resins include Amberlite IR 120 and equivalent resins.

Alternatively ion-replacement may be effected by reaction of the protonated amine cation with a salt-precusor compound, which may be a base, for example a carbonate, bicarbonate or hydroxide of a pharmaceutically acceptable alkali or alkaline earth metal, or a salt of an organic carboxylic acid with a pharmaceutically acceptable cation such as an alkali or alkaline earth metal, for example a salt of an alkanoic acid of formula (IV):

R¹⁰-CO₂H (IV)

wherein R¹⁰ is an alkyl group, containing for example from 1 to 20 carbon atoms, preferably from 1 to 8 carbon atoms. Examples of suitable salts include the acetate, propionate or ethylhexanoate salts, potassium 2-ethylhexanoate and sodium 2-ethylhexanoate being preferred. Typically the salt of clavulanic acid with amine (II-a) in solution may be reacted with a salt of an alkali metal with acid (IV) in solution or suspension in a suitable solvent, which may for example be an organic solvent, water, or a mixture of water and an organic solvent such as isopropanol. Suitably solutions of the salt of clavulanic acid with the amine (II-a) and of the salt-precursor compound may be mixed, and the pharmaceutically acceptable salt allowed to crystallise. Suitably the reaction may be carried out of a temperature below ambient, e.g. 0 to 15° C, e.g. 0 to 10°C, suitably 0 to 0.5°C. Suitably if the salt of clavulanic acid with the amine (II-a) is formed in aqueous solution it may be precipitated out by admixing the aqueous solution with excess acetone.

Suitable methods of esterification include:
a) the reaction of the salt of clavulanic acid with the amine (II) with a compound of the formula Q-R¹¹ wherein Q is a readily displaceable group and R¹¹ is an organic group;
b) the reaction of the salt of clavulanic acid with the amine (II) with an alcohol or thiol in the presence of a condensation promoting agent such as carbodiimide; and
c) the reaction of the salt of clavulanic acid with amine (II) with a diazo compound.

The foregoing processes extend to cover those aspects wherein the salt of clavulanic acid with amine (II-a) is first converted to clavulanic acid or another salt thereof and subsequently is converted to the desired ester. Further details of esterification methods are disclosed in GB 1508977 and 1508978. The present invention enables salts and esters of clavulanic acid to be more readily obtained in pure form than operation of the processes of GB1508977 and 1543563.

The invention will now be described by way of example only.

### Example 1

The amine was mixed with clavulanic acid in solution in THF and rapid crystallisation to form a solid salt was observed.

| **Amine** | **Comments** | **Stability of Salt** |
|---|---|---|
| triethylene tetramine | pale yellow solid | low assay |

### Reference Example 2

An impure solution of clavulanic acid in ethyl acetate (1L, 10.14 µg/ml) obtained by extraction of an *S. clavuligerus* fermentation broth with ethyl acetate and some pre-purification by ion-exchange, was mixed with acetone (600ml), and then treated with benzathine (6.15g) in ethyl acetate (55 ml). The mixture started to crystallise as the addition of benzathine neared completion. Methyl isobutyl ketone (200 ml) was then added to make precipitation more complete. The crystals were filtered off and was washed with acetone (3 x 100 ml). Yield = 11.5g, 67.0% recover.

### Reference Example 3.

The procedure of Reference Example 2 was repeated using the impure, wet (ca. 1% water), solution of clavulanic acid in ethyl acetate. To separate 1L batches of this solution was added with stirring an excess of neat ethylene diamine, NN'-diethylethylene diamine, and NN'-diisopropylethylenediamine, the quantity of each amine being in excess of the amount needed to form a diammonium salt with the clavulanic acid. After continued stirring a precipitate of the salt formed. A further crop of crystals was obtained by addition of excess acetone. The precipitated diammonium salt was filtered off and washed with acetone.

The crystals of each of these diammonium salts so formed were converted to potassium clavulanate by dissolution of the salt in the minimum quantity of water, followed by the addition of a solution of an excess of potassium 2-ethylhexanoate in isopropyl alcohol. After continued stirring a precipitate of potassium clavulanate formed and was filtered, washed with isopropyl alcohol and dried.

## Claims

1. Salts of clavulanic acid with polyamines of the general formula (II-a) as a solid precipitate 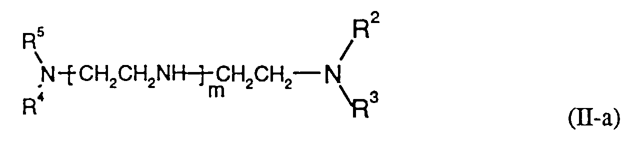 wherein R⁴ and R⁵ are independently hydrogen, C₁₋₆-alkyl, amino-substituted C₁₋₆ alkyl or substituted amino-substituted C₁₋₆ alkyl, and R² and R³ are independently selected from hydrogen, C₁₋₆-alkyl, amino- or hydroxy-substituted C₁₋₆ alkyl or substituted amino-substituted C₁₋₆ alkyl and m is zero or an integer 1 to 5 provided that when m is zero alkylenediammonium diclavulanates, with the exclusion of compounds, wherein R⁴ and R⁵ are hydrogen, C₁₋₆ alkyl or an aminoalkyl group having 2 to 4 carbon atoms, which is optionally substituted by an N-alkyl or N,N dialkyl group having 1 to 4 carbon atoms and wherein R² and R³ are hydrogen, C₁₋₆ alkyl, a hydroxyalkyl group having 2 to 4 carbon atoms or an aminoalkyl group having 2 to 4 carbon atoms, which is optionally substituted by an N-alkyl or N,N dialkyl group having 1 to 4 carbon atoms.

2. Salts according to claim 1 in which m is zero.

3. A process for preparing potassium clavulanate which comprises converting a salt as defined in claim 1 to potassium clavulanate.

## Patentansprüche

1. Salze der Clavulausäure mit Polyaminen der allgemeinen Formel (II-a) als ein festes Präzipitat 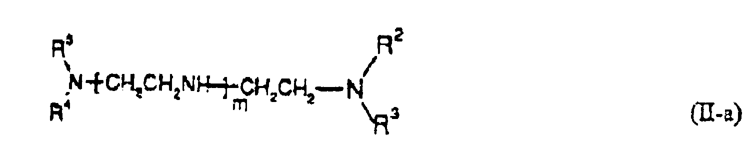 wobei die Reste R⁴ und R⁵ unabhängig Wasserstoffatome, C₁₋₆-Alkylreste, amino-substituierte C₁₋₆-Alkylreste oder substituierte amino-substituierte C₁₋₆-Alkylreste sind, und die Reste R² und R³ unabhängig ausgewählt sind aus Wasserstoffatomen, C₁₋₆-Alkylresten, amino- oder hydroxy-substituierten C₁₋₆-Alkylresten oder substituierten amino-substituierten C₁₋₆-Alkylresten und m den Wert 0 hat oder eine ganze Zahl mit dem Wert 1 bis 5 ist, mit der Maßgabe, daß falls m den Wert 0 hat, Alkylendiammonium-diclavulanate ausgenommen sind, in denen die Reste R⁴ und R⁵ Wasserstoffatome, C₁₋₆-Alkylreste, oder ein Aminoalkylrest mit 2 bis 4 Kohlenstoffstomen sind, der gegebenenfalls mit einem N-Alkylrest oder N,N-Dialkylrest mit 1 bis 4 Kohlenstoffatomen substituiert ist, und in denen die Reste R² und R³ Wasserstoffatome, C₁₋₆-Alkylreste, ein Hydroxyalkylrest mit 2 bis 4 Kohlenstoffatomen oder ein Aminoalkylrest mit 2 bis 4 Kohlenstoffatomen sind, der gegebenenfalls mit einem N-Alkylrest oder N,N-Dialkylrest mit 1 bis 4 Kohlenstoffatomen substituiert ist.

2. Salze gemäß Anspruch 1, wobei m den Wert 0 hat.

3. Ein Verfahren zur Herstellung von Kaliumclavulanat, das das Umwandeln eines Salzes, wie in Anspruch 1 definiert, in Kaliumclavulanat umfaßt.

## Revendications

1. Sels d'acide clavulanique avec des polyamines de formule générale (II-a), sous forme d'un précipité solide 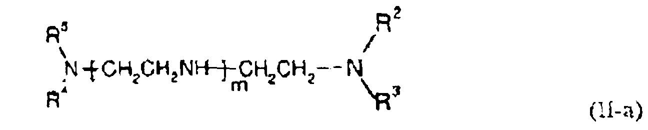 formule dans laquelle R⁴ et R⁵ représentent indépendamment de l'hydrogène, un groupe alkyle en C₁ à C₆, un groupe alkyle en C₁ à C₆ à substituant amino ou un groupe alkyle en C₁ à C₆ à substituant amino et substitué et dans laquelle R² et R³ sont choisis indépendamment parmi l'hydrogène, un groupe alkyle en C₁ à C₆, un groupe alkyle en C₁ à C₆ à substituant amino ou hydroxy, ou un groupe alkyle en C₁ à C₆ à substituant amino et substitué et dans laquelle m est égal à zéro ou à un nombre entier compris entre 1 et 5, sous réserve que lorsque m est égal à zéro, les diclavulanates d'alkylène-diammonium, dans lesquels R⁴ et R⁵ représentent de l'hydrogène, un groupe alkyle en C₁ à C₆ ou un groupe aminoalkyle ayant 2 à 4 atomes de carbone qui est facultativement substitué par un groupe N-alkyle ou N,N-dialkyle ayant 1 à 4 atomes de carbone et dans lesquels R² et R³ représentent de l'hydrogène, un groupe alkyle en C₁ à C₆ un groupe hydroxyalkyle ayant 2 à 4 atomes de carbone ou un groupe aminoalkyle ayant 2 à 4 atomes de carbone facultativement substitué par un groupe N-alkyle ou N,N-dialkyle ayant 1 à 4 atomes de carbone, soient exclus.

2. Sels suivant la revendication 1, dans lesquels m est égal à zéro.

3. Procédé de préparation de clavulanate de potassium qui comprend la transformation d'un sel tel que défini dans la revendication 1, en clavulanate de potassium.
